(19) Europäisches Patentamt
European Patent Office
Office européen des brevets

(11) EP 4 328 247 A1

(12) EUROPEAN PATENT APPLICATION

(43) Date of publication:
**28.02.2024 Bulletin 2024/09**

(21) Application number: **22192188.5**

(22) Date of filing: **25.08.2022**

(51) International Patent Classification (IPC):
***C08F 220/18*** (2006.01) ***C09J 133/06*** (2006.01)

(52) Cooperative Patent Classification (CPC):
(C-Sets available)
**C09J 133/066; C08F 212/08; C08F 220/1804**

(Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Henkel AG & Co. KGaA**
**40589 Düsseldorf (DE)**

(72) Inventors:
- **Brandt, Adrian**
  **45219 Essen (DE)**
- **Kropff, Marlo**
  **40599 Düsseldorf (DE)**
- **Ernst, Philipp**
  **42697 Solingen (DE)**

# (54) CONDUCTIVE PRESSURE SENSITIVE ADHESIVE

(57) The present invention relates to a conductive pressure sensitive adhesive comprising a copolymer obtained from a reaction mixture comprising an acrylic monomer mixture and a special styrene derivative, a process for the production of said conductive pressure sensitive adhesive composition and its use.

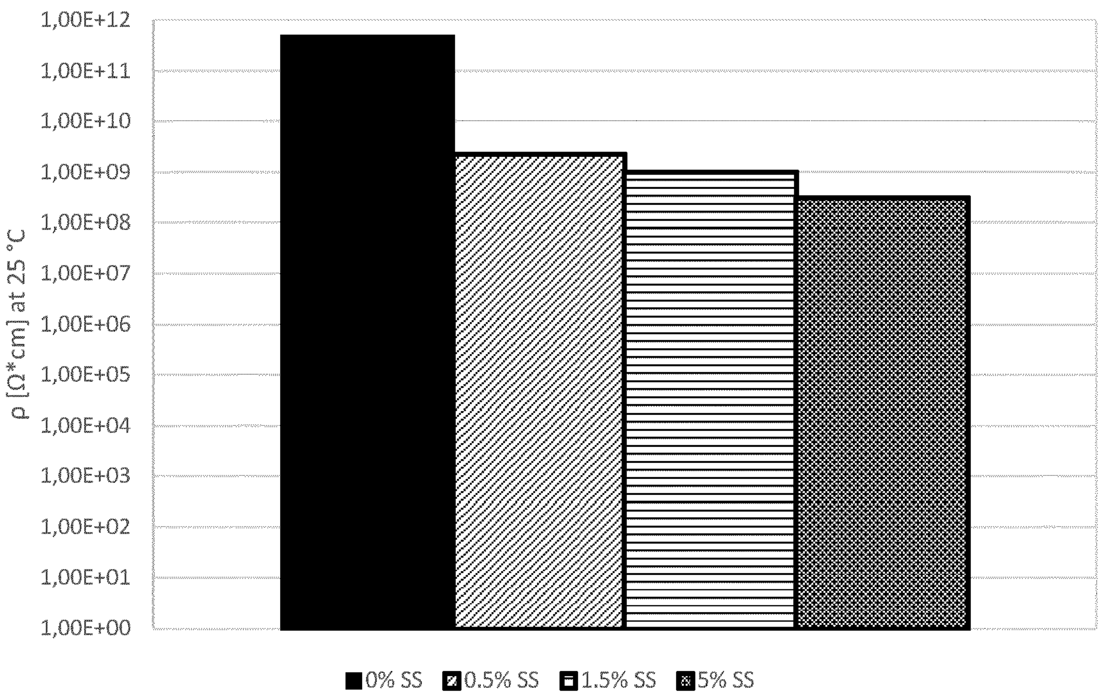


Figure 1

EP 4 328 247 A1

(52) Cooperative Patent Classification (CPC): (Cont.)

C-Sets
**C08F 220/1804, C08F 212/30, C08F 220/1808,**
**C08F 220/20**

## Description

The present invention relates to a conductive pressure sensitive adhesive comprising a copolymer obtained from a reaction mixture comprising an acrylic monomer mixture and a special styrene derivative, a process for the production of said conductive pressure sensitive adhesive composition and its use.

Conductive adhesives are primarily used for electronics, especially in technical fields such as aerospace and automotive. In recent years conductive adhesives have also become of interest for electrodes in medical and diagnostic applications such as electrocardiography (ECG), electroencephalography (EEG) and electromyography (EMG).

In this regard US 2018/086948 provides an adhesive composition comprising a resin and an electro-conductive material, wherein the electro-conductive material is an ammonium salt of fluorosulfonic acid having 5 or more carbon atoms shown by the following general formula (1): $(R^1\text{-X-Z-}SO_3^-)_n\ M^{n+}$, wherein, $R^1$ represents a linear, branched, or cyclic monovalent hydrocarbon group having 1 to 40 carbon atoms and optionally substituted by a heteroatom or optionally interposed by a heteroatom; X represents any of a single bond, an ether group, an ester group, and an amide group; Z represents a linear or branched alkylene group having 2 to 4 carbon atoms, containing 1 to 6 fluorine atoms, and optionally containing a carbonyl group; $M^{n+}$ represents a cation having one or two ammonium cation structures; and "n" is 1 when the number of the ammonium cation structure contained in the $M^{n+}$ is one, or is 2 when the number of the ammonium cation structure contained in the $M^{n+}$ is two.

WO 2020/178218 discloses an ionically conductive pressure sensitive adhesive composition comprising a) a (meth)acrylate resin comprising at least 10% of a (meth)acrylate monomer comprising OH-group by weight of the total weight of the (meth)acrylate resin; and b) an ionic liquid.

Despite the efforts already made, there is still the need for conductive pressure sensitive adhesive which can be applied to detect bio-signals such as heart rate or brain waves, especially in long-term monitoring. Another aspect is the problem of sustainability as the materials can usually only used once.

In order to address said needs, the present invention provides a conductive pressure sensitive adhesive based on materials obtainable from renewal sources. In this regard, a first aspect of the present invention is a conductive pressure sensitive adhesive composition comprising a copolymer obtained from a reaction mixture comprising

a) a monomeric acrylic mixture; and
b) a compound of Formula (I):

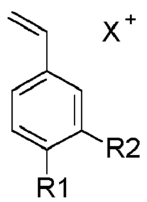

(I)

wherein $R^1$ is either a sulfate group ($OSO_3^-$), a sulfonate group ($SO_3^-$), or a hydrogen atom (H); $R^2$ is a sulfate group, a sulfonate group, an alkoxy group or a hydrogen atom,
and wherein $X^+$ is a counter ion with the proviso that either $R^1$ or $R^2$ is an ionic group.

Within the course of the present invention, it was found that the conductive pressure sensitive adhesive composition of the present invention provides improved ion conductivity and increased peel strength, even in the absence of an ionic liquid as commonly employed. The inventive composition further has the advantage that the compound of Formula (I) can be incorporated into the acrylate polymeric backbone.

The conductive pressure sensitive adhesive composition according to the present invention can advantageously be used as a dry film, which offers a solution for long-term monitoring of biosignals by acting as a functional contact between electrode and skin. In contrast to gel-type electrodes currently in the market it does not have the draw-back of drying out or causing skin irritation. Furthermore, the impedance of the conductive pressure sensitive adhesive composition according to the present invention is very low without any addition of water.

The counter ion $X^+$ is preferably selected from the group of $NR^3R^4R^5R^{6+}$, with each of $R^3$, $R^4$, $R^5$ and $R^6$ being independently selected from $C_1$-$C_8$-alkyl, preferably $C_2$-$C_6$-alkyl, 1-ethyl-3-methylimidazolium, choline, pyridinium, pyrrolidinium and mixtures thereof. In an especially preferred embodiment, is $X^+$ is $N(C_4H_9)_4^+$.

With respect to the compound of Formula (I), $R^1$ is preferably a sulfate group and $R^2$ is an alkoxy group or a hydrogen atom. In an especially preferred embodiment, $R^1$ is a sulfate group and $R^2$ is a hydrogen atom.

The alkoxy group of the compound of Formula (I), if present, is preferably selected from the group of OMe,

OEt, OPr and OBu, with preference given to OMe.

In an especially preferred embodiment, the compound of Formula (I) is the following:

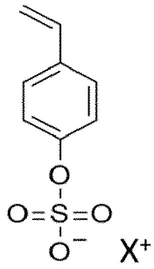

(I)

with $X^+$ defined as above.

The conductive pressure sensitive adhesive comprises a copolymer obtained from a reaction mixture comprising a monomeric acrylic mixture. In a preferred embodiment, the monomeric acrylic mixture comprises at least one of the following: methyl methacrylate , hydroxyethyl acrylate, hydroxypropyl acrylate, hydroxybutyl acrylate, n-butyl acrylate, 2-ethylhexyl acrylate, acrylic acid, C1-C18 alkyl (meth)acrylate, (meth)acrylamide, vinyl acetate, N-vinyl caprolactame, acrylonitrile, vinyl ether, benzyl (meth)acrylate, cyclohexyl (meth)acrylate, glycidyl (meth)acrylate and mixtures thereof, preferably formed from the monomers selected from the group consisting of hydroxyethyl acrylate, methyl methacrylate, n-butyl acrylate, 2-ethylhexyl acrylate and mixtures thereof.

Especially preferred is an embodiment of the present invention wherein the monomeric acrylic mixture comprises at least one, preferably all, of the following 2-ethylhexyl acrylate (abbreviation: 2-EHA), methyl methacrylate (abbreviation: MMA), 2-hydroxyethyl acrylate (abbreviation: 2-HEA), n-butyl acrylate (abbreviation: BA).

In a preferred embodiment of the conductive pressure sensitive adhesive composition according to the invention, the reaction mixture contains 2-EHA in an amount of 1 to 20 wt.-%, preferably 2 to 7 wt.-% in the reaction mixture, based on the total weight of the reaction mixture.

In a preferred embodiment of the conductive pressure sensitive adhesive composition according to the invention, the reaction mixture contains MMA in an amount of 1 to 7 wt.-%, preferably 2 to 5 wt.-% in the reaction mixture, based on the total weight of the reaction mixture.

In a preferred embodiment of the conductive pressure sensitive adhesive composition according to the invention, the reaction mixture contains 2-HEA in an amount of 15 to 40 wt.-%, preferably 20 to 35 wt.-% in the reaction mixture, based on the total weight of the reaction mixture.

In a preferred embodiment of the conductive pressure sensitive adhesive composition according to the invention, the the reaction mixture contains BA in an amount of 40 to 80 wt.-%, preferably 55 to 65 wt.-% in the reaction mixture, based on the total weight of the reaction mixture.

In a preferred embodiment of the conductive pressure sensitive adhesive composition according to the invention, the compound of formula (I) is contained in an amount of 0.1 to 50 wt.-%, preferably 0.2 to 30 wt.-%, especially 0.5 to 10 wt.-%, in the reaction mixture, based on the total weight of the reaction mixture.

Within the course of the present invention, it was surprisingly found that addition of an ionic liquid to the conductive pressure sensitive adhesive composition enables faster build-up of peel strength over time compared with compositions merely comprising either an ionic liquid or the compound of Formula (I). Therefore, in a preferred embodiment, the conductive pressure sensitive adhesive composition further comprises an ionic liquid.

The ionic liquid optionally comprised in conductive pressure sensitive adhesive composition is preferably a non-toxic, non-irritating ionic liquid leading to ionic conductivity.

More specifically, the ionic liquid is selected from the group consisting of imidazolium acetates, imidazolium sulfonates, imidazolium chlorides, imidazolium sulphates, imidazolium phosphates, imidazolium thiocyanates, imidazolium dicyanamides, imidazolium benzoates, imidazolium triflates, choline triflates, choline saccharinate, choline sulfamates, pyridinium acetates, pyridinium sulfonates, pyridinium chlorides, pyridinium sulphates, pyridinium phosphates, pyridinium thiocyanates, pyridinium dicyanamides, pyridinium benzoates, pyridinium triflates, pyrrolidinium acetates, pyrrolidinium sulfonates, pyrrolidinium chlorides, pyrrolidinium sulphates, pyrrolidinium phosphates, pyrrolidinium thiocyanates, pyrrolidinium dicyanamides, pyrrolidinium benzoates, pyrrolidinium triflates, phosphonium acetates, phosphonium sulfonates, phosphonium chlorides, phosphonium sulphates, phosphonium phosphates, phosphonium thiocyanates, phosphonium dicyanamides, phosphonium benzoates, phosphonium triflates, sulfonium acetates, sulfonium sulfonates, sulfonium chlorides, sulfonium sulphates, sulfonium phosphates, sulfonium thiocyanates, sulfonium dicyanamides, sulfonium benzoates, sulfonium triflates, ammonium acetates, ammonium sulfonates, ammonium chlorides, ammonium sulphates, ammonium phosphates, ammonium thiocyanates, ammonium dicyanamides, ammonium benzoates, ammonium triflates and mixtures thereof.

Preferably, said ionic liquid is selected from the group consisting of 1-ethyl-3-methylimidazolium acetate, 1-ethyl-3-methylimidazolium methane sulfonate, 1-ethyl-3-methylimidazolium trifluoromethane sulfonate, 1-ethyl-3-methylimidazolium chloride, 1-ethyl-3-methylimidazolium ethyl sulphate, 1-ethyl-3-methylimidazolium diethylphosphate, 1-ethyl-3-methylimidazolium thiocyanate, 1-ethyl-3-methylimidazolium dicyanamide, 1-ethyl-3-methylimidazolium benzoate, choline trifluoromethanesulfonate, choline saccharinate, choline acesulfamate, choline N-cyclohexylsulfamate, tris(2-hydroxyethyl)methylammonium methyl sulphate, 1-ethyl-3-methylimidazolium tetrafluoroborate, 1-allyl-3-methylimidazolium bis(trifluoromethylsulfonyl)imide, choline acetate and mixtures thereof.

More preferably, the ionic liquid is selected from the group consisting of 1-ethyl-3-methylimidazolium benzoate, 1-ethyl-3-methylimidazolium tetrafluoroborate, 1-ethyl-3-methylimidazolium methane sulfonate, 1-ethyl-3-methylimidazolium chloride, 1-ethyl-3-methylimidazolium trifluoromethane sulfonate, choline trifluoromethane sulfonate, 1-ethyl-3-methylimidazolium acetate, choline acetate, 1-ethyl-3-methylimidazolium diethylphosphate, 1-allyl-3-methylimidazolium bis(trifluoromethylsulfonyl)imide, 1-ethyl-3-methylimidazolium ethyl sulphate, 1-ethyl-3-methylimidazolium thiocyanate, 1-ethyl-3-methylimidazolium dicyanamide, choline saccharinate, choline acesulfamate, and mixture thereof.

In one embodiment of the conductive pressure sensitive adhesive composition, two or more ionic liquids are used, in this embodiment said ionic liquids are selected from the group consisting of 1-ethyl-3-methylimidazolium acetate, 1-ethyl-3-methylimidazolium methane sulfonate, 1-ethyl-3-methylimidazolium trifluoromethane sulfonate, 1-ethyl-3-methylimidazolium chloride, 1-ethyl-3-methylimidazolium ethyl sulphate, 1-ethyl-3-methylimidazolium diethylphosphate, 1-ethyl-3-methylimidazolium thiocyanate, 1-ethyl-3-methylimidazolium dicyanamide, 1-ethyl-3-methylimidazolium benzoate, choline trifluoromethanesulfonate, choline saccharinate, choline acesulfamate, choline N-cyclohexylsulfamate, tris(2-hydroxyethyl)methylammonium methyl sulphate, 1-ethyl-3-methylimidazolium tetrafluoroborate, 1-allyl-3-methylimidazolium bis(trifluoromethylsulfonyl)imide, choline acetate; preferably two or more ionic liquids are selected from the group consisting of 1-ethyl-3-methylimidazolium benzoate, 1-ethyl-3-methylimidazolium tetrafluoroborate, 1-ethyl-3-methylimidazolium methane sulfonate, 1-ethyl-3-methylimidazolium chloride, 1-ethyl-3- methylimidazolium trifluoromethane sulfonate, choline trifluoromethane sulfonate, 1-ethyl-3-methylimidazolium acetate, choline acetate, 1-ethyl-3-methylimidazolium diethylphosphate, 1-allyl-3-methylimidazolium bis(trifluoromethylsulfonyl)imide, 1-ethyl-3-methylimidazolium ethyl sulphate, 1-ethyl-3-methylimidazolium thiocyanate, 1-ethyl-3-methylimidazolium dicyanamide, choline saccharinate, choline acesulfamate.

In an especially preferred embodiment, the ionic liquid is selected from the group consisting of 1-ethyl-3-methylimidazolium trifluoromethanesulfonate, 1-ethyl-3-methylimidazolium acetate, 1-ethyl-3-methylimidazolium methanesulfonate, 1-ethyl-3-methylimidazolium chloride, 1-ethyl-3-methylimidazolium sulfate, 1-ethyl-3-methylimidazolium diethylphosphate, 1-ethyl-3-methylimidazolium thiocyanate, 1-ethyl-3-methylimidazolium dicyanamide, 1-ethyl-3-methylimidazolium benzoate, 1-ethyl-3-methylimidazolium tetrafluoroborate, choline trifluoromethanesulfonate, choline saccharinate, choline acesulfamate, choline N-cyclohexylsulfamate, tris(2.hydroxyethyl)methylammonium methyl sulfate, choline acetate and mixtures thereof, and more preferably selected from the group consisting of 1-ethyl-3-methylimidazolium trifluoromethanesulfonate, 1-ethyl-3-methylimidazolium acetate, 1-ethyl-3-methylimidazolium methanesulfonate, 1-ethyl-3-methylimidazolium chloride, 1-ethyl-3-methylimidazolium sulfate, 1-ethyl-3-methylimidazolium diethylphosphate, 1-ethyl-3-methylimidazolium thiocyanate, 1-ethyl-3-methylimidazolium dicyanamide, 1-ethyl-3-methylimidazolium benzoate, 1-ethyl-3-methylimidazolium tetrafluoroborate, choline trifluoromethanesulfonate, choline saccharinate, choline acesulfamate, choline acetate, and mixtures thereof.

Suitable commercially available ionic liquids for use in the present invention include, but are not limited to Basionics ST80, Basionics Kat1 , Basionics BC01 , Basionics VS1 1 , Basionics VS03, and Efka IO 6785, all from BASF.

The ionic liquid is preferably comprised in the conductive pressure sensitive adhesive composition in an amount of from 0.1 to 35% by weight of the total weight of the composition, preferably from 0.5 to 25%, and more preferably from 1 to 15%.

It was surprisingly found that the best performance of the conductive pressure sensitive adhesive composition was achieved if a mixture of acrylic monomers was used to obtain the copolymer. Therefore, in a preferred embodiment, the conductive pressure sensitive adhesive composition the composition comprises a copolymer obtained from a reaction mixture comprising:

a. 2-EHA in an amount of 1 to 20 wt.-%, preferably 2 to 7 wt.-%
b. MMA in an amount of 1 to 7 wt.-%, preferably 2 to 5 wt.-%
c. 2-HEA in an amount of 15 to 40 wt.-%, preferably 20 to 35 wt.-%
d. BA in an amount of 40 to 80 wt.-%, preferably 55 to 65 wt.-%
e. compound of Formula (I) in an amount of 0.1 to 50 wt.-%, preferably 0.2 to 30 wt.-%, especially 0.5 to 10 wt.-%,

based on the total weight of the reaction mixture, respectively; and
an ionic liquid in the amount of from 0.1 to 35 wt.-%, preferably from 0.5 to 25 wt.-%, and more preferably from 1 to 15

wt.-%, based on the total weight of the conductive pressure sensitive adhesive composition, respectively.

In another aspect, the present invention refers to a process for the production of the conductive pressure sensitive adhesive composition according to the invention, the process comprising the following steps:

a) providing an initial and a feed solution of a reaction mixture comprising a monomeric acrylic mixture and a compound of formula (I);
b) providing an initial and a feed solution of an initiator solution;
c) adding the initial initiator solution to the initial solution of the reaction mixture at an ambient temperature;
d) heating the reaction mixture of step c) to temperatures between 60 °C and 120 °C, preferably to temperatures between 70 °C and 90 °C;
e) continuously adding the feed solution of the reaction mixture and feed solution of the initiator solution.

In a preferred embodiment, step e) of the inventive process is carried out over a period of 2 to 8 hours, preferably over 3 to 5 hours.

The initiator employed may be any suitable component for initiating the polymerization of acrylic and styrenic monomers. Preferably, the initiator is selected from the group consisting of 2,2'-azobisisobutyronitril, 1,1'-Azobis(cyclohexanecarbonitrile), Dimethyl 2,2'-azobis(2-methylpropionate), 2,2'-Azobis(2,4-dimethylvaleronitrile),2,2'-Azobis(2-methylbutyronitrile), Benzoyl peroxide, tert-butyl hydroperoxide, tert-butyl peracetate, tert-butyl peroxide, tert-butyl peroxybenzoate, tert-butylperoxy isopropyl carbonate, cumene hydroperoxide, cyclohexanone peroxide, dicumyl peroxide, lauroyl peroxide. Special preference is given to 2,2'-azobisisobutyronitril (AIBN) as initiator.

The conductive pressure sensitive adhesive composition of the present invention is particularly suitable to be brought into contact with human skin and for employment in long-term monitoring of body functions such as heart rate or brain waves. Therefore, a further object of the present invention is the use of the conductive pressure sensitive adhesive composition according to the invention in skin applications as a contact medium as part of electrodes measuring bio-signals from the skin, athletic tape, transdermal drug patches, permanent adhesive tapes for industrial use and permanent adhesive tapes for construction work. In addition, the conductive pressure sensitive adhesive is preferably used in applications for safety labels for power equipment, masking tapes, price marking labels and/or electrochemical debonding.

The present invention will be described in more detail with reference to the examples below. However, these are in no way to be understood as limiting the scope and spirit of the invention.

**Example 1:**

i) Preparation of conductive pressure sensitive adhesive (PSA) compositions

The compositions of table 1 were prepared according in the following manner:
An initiator solution was added to an initial monomeric acrylic mixture containing of 2-EHA, MMA, 2-HEA, BA and the compound of formula (I) as depicted below and heated to 80 °C while stirring. Additional solution of monomeric acrylic mixture and initiator solution were continuously added. The mixture was then cooled to room temperature and diluted with ethanol, isopropanol and ethyl acetate to obtain the final polymer solution.

The following compound was employed as compound of Formula (I) which is in the following abbreviated by SS (styrene sulfate):

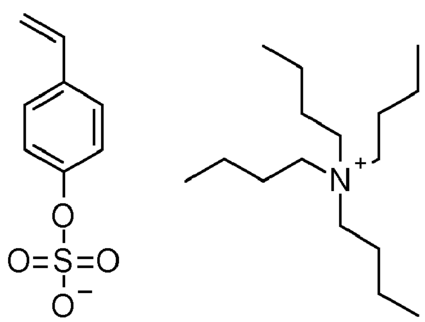

Table 1:

| Batch | acrylic mixture (2-EHA, MMA, 2-HEA, BA) [wt.-%] | SS [wt.-%] |
|---|---|---|
| A* | 100 | 0 |
| B | 99.5 | 0.5 |
| C | 98,5 | 1.5 |
| D | 95 | 5 |
| *) comparative | | |

ii) Preparation of PSA films and procedure for impedance measurements

The PSA films for property testing were prepared by casting a film of the respective polymer solution on silicone paper. After application, the film was dried in an oven at 115 °C for 5 min and covered with silicone paper. Film thickness was adjusted to obtain films with a coating weight $\rho_A$ of 30g/m$^2$. For each polymer solution, two 2 x 2 cm testing strips were applied. The two substrates were then glued together at an angle of 180° and impedance measurements at a frequency range 9*10$^6$ Hz $\leq v \leq$ 0.1 Hz were performed with a potentiostat. The resistivity $\rho$ was calculated as shown in equation 1 with the measured impedance Z, overlap area A and layer thickness d.

$$\rho = \frac{Z \cdot A}{d} \qquad (1)$$

iii) Decrease in resistivity by the compound of Formular (I)

Data gathered in impedance measurements is listed in Table 2 and visualized in Figure 1. The resistivity of the PSA decreases with increasing content of the compound of Formular (I). As conductivity is defined as the reciprocal resistivity, the conductivity increases with increasing content of the compound of Formula (I). This can be an advantage for applications in the electronics industry or the medical industry, such as ECG medical patches. As is apparent from the data of Figure 1, resistivity decreases with increasing SS content.

Table 2:

| w (SS) wt%. | ρ 10$^8$ Ω*cm |
|---|---|
| 0 | 4825 |
| 0.5 | 22.74 |
| 1.5 | 9.984 |
| 5 | 3.101 |

**Example 2:**

i) Preparation of polymer-ionic liquid blends

Polymer solutions were prepared as described in example 1. The ionic liquid (IL) 1-Ethyl-3-methylimidazolium triflate was added to prepare polymer-ionic liquid blends. Blends with 5 wt% or 10 wt% of the ionic liquid with regard to the solid content of the polymer solution were prepared.

ii) Preparation of PSA films for peel tests

The PSA films for property testing were prepared by applying the respective polymer solution on an etched polyethylene foil. After application, the film was dried in an oven at 115 °C for 5 min and covered with silicone paper. Film thickness was adjusted to obtain films with a coating weight $\rho_A$ of 30g/m$^2$.

iii) Preparation of 180 ° peel test samples and procedure

180° peel tests were performed according to protocol FTM1 defined by European association for the self-adhesive label industry (FINAT).

Data gathered in 180° peel tests of the polymer-ionic liquid blends is listed in Table 3 and visualized in Figure 2. Without IL and with 5 wt% IL, the presence of a compound of Formula (I) in the polymer increases the adhesion of the PSA on stainless steel and glass substrates. Peel strength increases with increasing content of a compound of Formula (I). An increase is desirable for high performance PSAs. Figure 2 summarizes the evaluation of 180° peel tests 24 hours after application on stainless steel (Figure 2a) and glass (Figure 2b). Tests were performed with crude polymer solutions (left) and polymer-ionic liquid blends with 5 wt% IL (right) with 0 (solid), 0.5 (diagonal), 1.5 (horizontal) and 5 wt% of a compound of Formula (I) (crossed). As is apparent from the data provided, peel strength increases with increasing content of a compound of Formula (I) on both substrates with and without IL added.

Table 3:

| Substrate | w (SS) wt%. | w (IL) wt%. | Peel strength [N/25 mm] |
|---|---|---|---|
| Stainless steel | 0 | 0 | 40.3 ± 0.5 |
| Stainless steel | 0.5 | 0 | 39 ± 4 |
| Stainless steel | 1.5 | 0 | 42.1 ± 0.8 |
| Stainless steel | 5 | 0 | 45 ± 2 |
| Stainless steel | 0 | 5 | 32.9 ± 0.9 |
| Stainless steel | 0.5 | 5 | 33 ± 2 |
| Stainless steel | 1.5 | 5 | 34.2 ± 0.3 |
| Stainless steel | 5 | 5 | 36.13 ± 0.02 |
| Glass | 0 | 0 | 40.1 ± 0.6 |
| Glass | 0.5 | 0 | 41 ± 2 |
| Glass | 1.5 | 0 | 40.0 ± 0.2 |
| Glass | 5 | 0 | 42.5 ± 0.2 |
| Glass | 0 | 5 | 29 ± 7 |
| Glass | 0.5 | 5 | 31 ± 3 |
| Glass | 1.5 | 5 | 33.4 ± 0.5 |
| Glass | 5 | 5 | 33.9 ± 0.5 |

iv) Synergistic effect of SS and IL on substrate wetting

Data gathered in 180° peel tests of polymer-ionic liquid blends is listed in Table 4 and visualized in Figure 3. If no comonomers of Formula (I) are present in the polymer, the adhesive needs time to reach its maximum strength independent from the addition of ionic liquid as shown by the significant difference in peel strength measured 20 minutes and 24 hours after application. The same observation is made for polymers containing 5 wt% of the compound of Formula (I), but no ionic liquid. However, if IL is added to a polymer with comonomers of Formula (I), peel strength 20 minutes after application increases significantly compared to the previously mentioned systems. Maximum peel strength is almost reached after 20 minutes. This observation shows a synergistic effect between the presence of comonomers of Formula (I) in the polymer and ILs which positively affects the wetting process in terms of a shortened application time.

With reference to Figure 3, evaluation data of 180° peel tests of polymer-ionic liquid blends on stainless steel are shown. Tests were performed 20 minutes (solid) and 24 hours (dotted) after application with 0, 5 and 10 wt% IL and 0 wt% of a compound of Formula (I) (Figure 3a) and 10 wt% of a compound of Formula (I) (Figure 3b). Without comonomers of Formula (I) and without IL, a significant difference in peel strength is observed over time. With comonomers of Formula (I) and IL, maximum peel strength is achieved after 20 minutes which shows the synergistic effect of the presence of comonomers of Formula (I) in the polymer and ILs on the wetting of the substrate by the polymer.

Table 4:

| Substrate | w (SS) wt%. | w (IL) wt%. | Time after application [h] | Peel strength [N/25 mm] |
|---|---|---|---|---|
| Stainless steel | 0 | 0 | 0.33 | 17.42 ± 0.05 |
| Stainless steel | 0 | 0 | 24 | 40.3 ± 0.5 |
| Stainless steel | 0 | 5 | 0.33 | 16 ± 4 |
| Stainless steel | 0 | 5 | 24 | 32.9 ± 0.9 |
| Stainless steel | 0 | 10 | 0.33 | 18 ± 5 |

(continued)

| Substrate | w (SS) wt%. | w (IL) wt%. | Time after application [h] | Peel strength [N/25 mm] |
|---|---|---|---|---|
| Stainless steel | 0 | 10 | 24 | 28 ± 2 |
| Stainless steel | 5 | 0 | 0.33 | 22 ± 2 |
| Stainless steel | 5 | 0 | 24 | 45 ± 2 |
| Stainless steel | 5 | 5 | 0.33 | 34.4 ± 0.7 |
| Stainless steel | 5 | 5 | 24 | 36.13 ± 0.02 |
| Stainless steel | 5 | 10 | 0.33 | 27.4 ± 0.3 |
| Stainless steel | 5 | 10 | 24 | 28 ± 2 |

The data provided not only shows the improved adhesion properties of the conductive pressure sensitive adhesive composition of the present invention, but also evidences the synergistic effects which can be achieved by combination with an ionic liquid.

## Claims

1. Conductive pressure sensitive adhesive composition comprising a copolymer obtained from a reaction mixture comprising

   a) a monomeric acrylic mixture; and
   b) a compound of Formula (I):

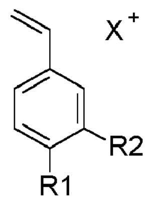

(I)

   wherein $R^1$ is either a sulfate group ($OSO_3^-$), a sulfonate group ($SO_3^-$), or a hydrogen atom (H); $R^2$ is a sulfate group, a sulfonate group, an alkoxy group or a hydrogen atom,
   and wherein $X^+$ is a counter ion with the proviso that either $R^1$ or $R^2$ is an ionic group.

2. . Conductive pressure sensitive adhesive composition according to claim 1, **characterized in that** $X^+$ is selected from the group of $NR^3R^4R^5R^{6+}$, with each of $R^3$, $R^4$, $R^5$ and $R^6$ being independently selected from $C_1$-$C_8$-alkyl, preferably $C_2$-$C_6$-alkyl, 1-ethyl-3-methylimidazolium, choline, pyridinium, pyrrolidinium and mixtures thereof.

3. . Conductive pressure sensitive adhesive composition according to claim 2, **characterized in that** $X^+$ is $N(C_4H_9)4^+$.

4. . Conductive pressure sensitive adhesive composition according to any of the forgoing claims, **characterized in that** the (meth)acrylic mixture comprises at least one of the following: methyl methacrylate , hydroxyethyl acrylate, hydroxypropyl acrylate, hydroxybutyl acrylate, n-butyl acrylate, 2-ethylhexyl acrylate, acrylic acid, C1-C18 alkyl (meth)acrylate, (meth)acrylamide, vinyl acetate, N-vinyl caprolactame, acrylonitrile, vinyl ether, benzyl (meth)acrylate, cyclohexyl (meth)acrylate, glycidyl (meth)acrylate and mixtures thereof, preferably formed from the monomers selected from the group consisting of hydroxyethyl acrylate, methyl methacrylate, n-butyl acrylate, 2-ethylhexyl acrylate and mixtures thereof.

5. . Conductive pressure sensitive adhesive composition according to any of the forgoing claims, **characterized in that** the compound of formula (I) is contained in an amount of 0.1 to 50 wt.-%, preferably 0.2 to 30 wt.-%, especially 0.5 to 10 wt.-%, in the reaction mixture, based on the total weight of the reaction mixture.

6. . Conductive pressure sensitive adhesive composition according to any of the forgoing claims, **characterized in that** reaction mixture contains 2-EHA in an amount of 1 to 10 wt.-%, preferably 2 to 7 wt.-% in the reaction mixture,

based on the total weight of the reaction mixture.

**7.** . Conductive pressure sensitive adhesive composition according to any of the forgoing claims, **characterized in that** the reaction mixture contains MMA in an amount of 1 to 7 wt.-%, preferably 2 to 5 wt.-% in the reaction mixture, based on the total weight of the reaction mixture.

**8.** . Conductive pressure sensitive adhesive composition according to any of the forgoing claims, **characterized in that** the reaction mixture contains 2-HEA in an amount of 15 to 40 wt.-%, preferably 20 to 35 wt.-% in the reaction mixture, based on the total weight of the reaction mixture.

**9.** . Conductive pressure sensitive adhesive composition according to any of the forgoing claims, **characterized in that** the reaction mixture contains BA in an amount of 40 to 80 wt.-%, preferably 55 to 65 wt.-% in the reaction mixture, based on the total weight of the reaction mixture.

**10.** . Conductive pressure sensitive adhesive composition according to any of the forgoing claims, **characterized in that** the pressure sensitive adhesive composition further comprises an ionic liquid, preferably selected from the group consisting of 1-ethyl-3-methylimidazolium trifluoromethanesulfonate, 1-ethyl-3-methylimidazolium acetate, 1-ethyl-3-methylimidazolium methanesulfonate, 1-ethyl-3-methylimidazolium chloride, 1-ethyl-3-methylimidazolium sulfate, 1-ethyl-3-methylimidazolium diethylphosphate, 1-ethyl-3-methylimidazolium thiocyanate, 1-ethyl-3-methylimidazolium dicyanamide, 1-ethyl-3-methylimidazolium benzoate, 1-ethyl-3-methylimidazolium tetrafluoroborate, choline trifluoromethanesulfonate, choline saccharinate, choline acesulfamate, choline N-cyclohexylsulfamate, tris(2.hydroxyethyl)methylammonium methyl sulfate, choline acetate and mixtures thereof, and more preferably selected from the group consisting of 1-ethyl-3-methylimidazolium trifluoromethanesulfonate, 1-ethyl-3-methylimidazolium acetate, 1-ethyl-3-methylimidazolium methanesulfonate, 1-ethyl-3-methylimidazolium chloride, 1-ethyl-3-methylimidazolium sulfate, 1-ethyl-3-methylimidazolium diethylphosphate, 1-ethyl-3-methylimidazolium thiocyanate, 1-ethyl-3-methylimidazolium dicyanamide, 1-ethyl-3-methylimidazolium benzoate, 1-ethyl-3-methylimidazolium tetrafluoroborate, choline trifluoromethanesulfonate, choline saccharinate, choline acesulfamate, choline acetate, and mixtures thereof.

**11.** . Conductive pressure sensitive adhesive composition according to claim 10, **characterized in that** the ionic liquid is present in an amount of 0.1 to 35 wt.-%, preferably 0.5 to 25 wt.-%, especially 1 to 15 wt.-%, based on the total weight of the pressure sensitive adhesive composition.

**12.** . Conductive pressure sensitive adhesive composition according to any of the forgoing claims, **characterized in that** the composition comprises a copolymer obtained from a reaction mixture comprising:

a) 2-EHA in an amount of 1 to 20 wt.-%, preferably 2 to 7 wt.-%
b) MMA in an amount of 1 to 7 wt.-%, preferably 2 to 5 wt.-%
c) 2-HEA in an amount of 15 to 40 wt.-%, preferably 20 to 35 wt.-%
d) BA in an amount of 40 to 80 wt.-%, preferably 55 to 65 wt.-%
e) compound of Formula (I) in an amount of 0.1 to 50 wt.-%, preferably 0.2 to 30 wt.-%, especially 0.5 to 10 wt.-%, based on the total weight of the reaction mixture; and

an ionic liquid in of 0.1 to 35 wt.-%, preferably 0.5 to 25 wt.-%, especially 1 to 15 wt.-%, based on the total weight of the pressure sensitive adhesive composition.

**13.** . Process for the production of a conductive pressure sensitive adhesive composition according to any of claims 1 to 12, the process comprising the following steps:

a) providing an initial and a feed solution of a reaction mixture comprising a monomeric acrylic mixture and a compound of formula (I);
b) providing an initial and a feed solution of an initiator solution;
c) adding the initial initiator solution to the initial solution of the reaction mixture at an ambient temperature;
d) heating the reaction mixture of step c) to temperatures between 60 °C and 120 °C, preferably to temperatures between 70 °C and 90 °C;
e) continuously adding the feed solution of the reaction mixture and feed solution of the initiator solution.

**14.** . Process according to claims 13, **characterized in that** the initiator is selected from the group consisting of 2,2'-azobisisobutyronitril, 1,1'-Azobis(cyclohexanecarbonitrile), Dimethyl 2,2'-azobis(2-methylpropionate), 2,2'-Azobis(2,4-dimethylvaleronitrile), 2,2'-Azobis(2-methylbutyronitrile), Benzoyl peroxide, tert-butyl hydroperoxide, tert-butyl peracetate, tert-butyl peroxide, tert-butyl peroxybenzoate, tert-butylperoxy isopropyl carbonate, cumene hydroperoxide, cyclohexanone peroxide, dicumyl peroxide, lauroyl peroxide.

**15.** . Use of a pressure sensitive adhesive composition according to any of claims 1 to 12 in skin applications as a contact medium as part of electrodes measuring bio-signals from the skin, athletic tape, transdermal drug patches, permanent adhesive tapes for industrial use and permanent adhesive tapes for construction work and/or for safety labels for power equipment, masking tapes, price marking labels and/or electrochemical debonding.

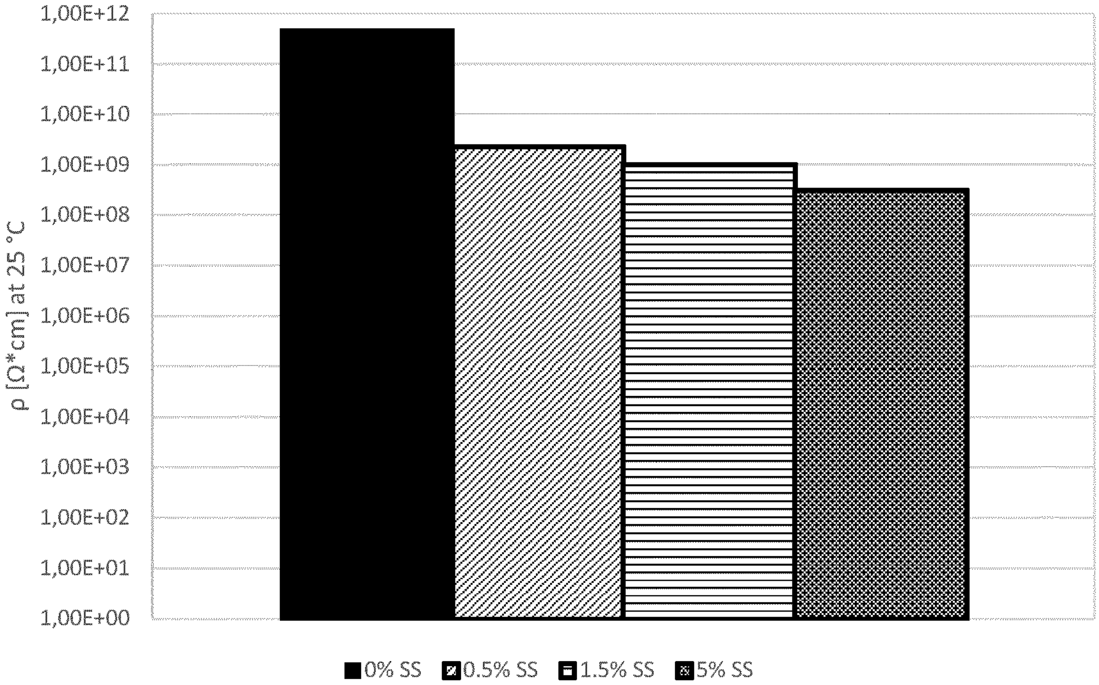


Figure 1

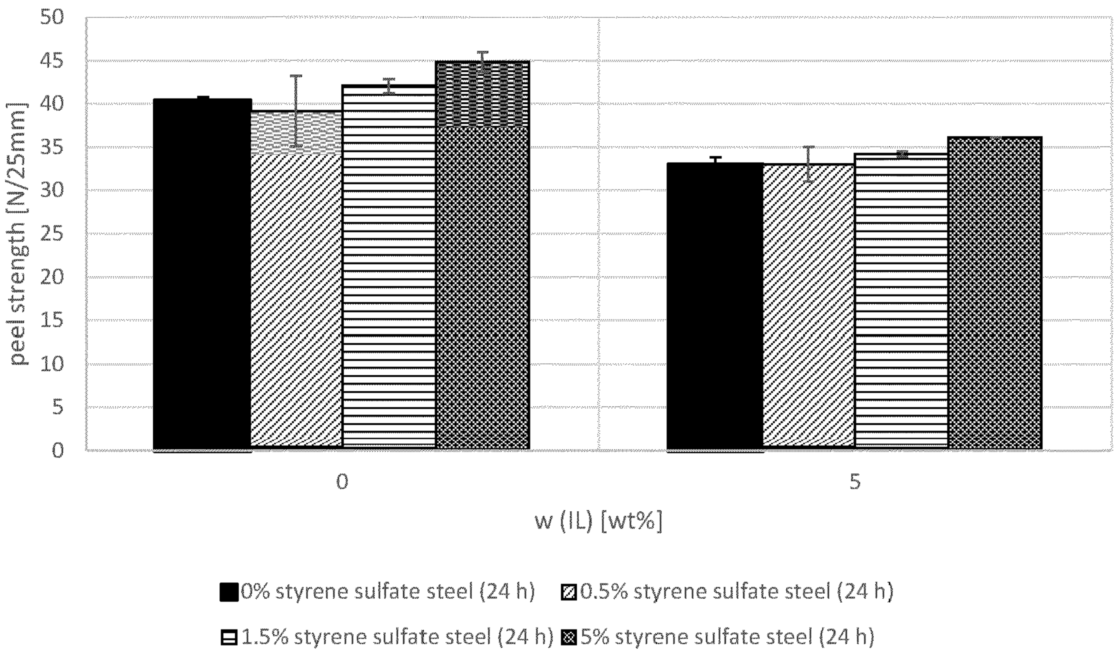


Figure 2a

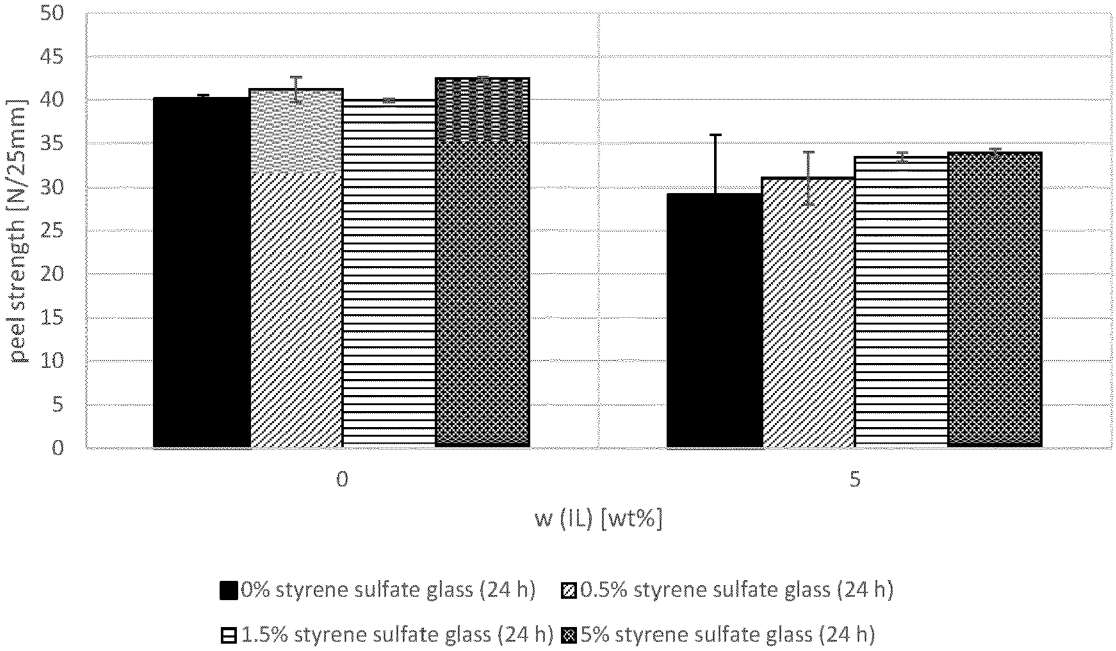


Figure 2b

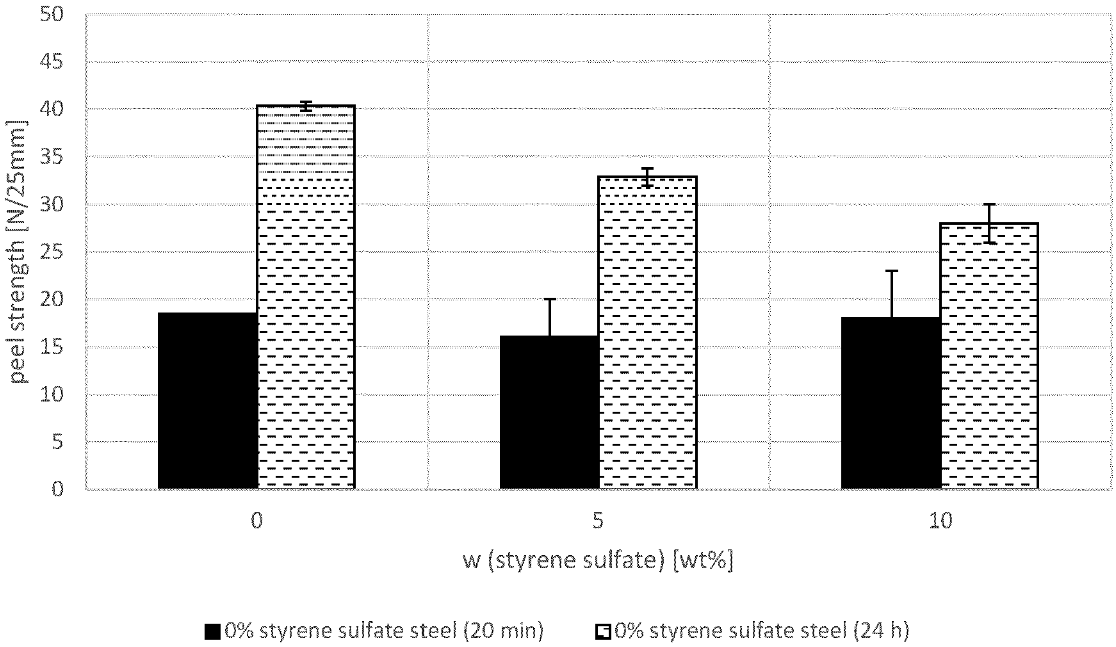


Figure 3a

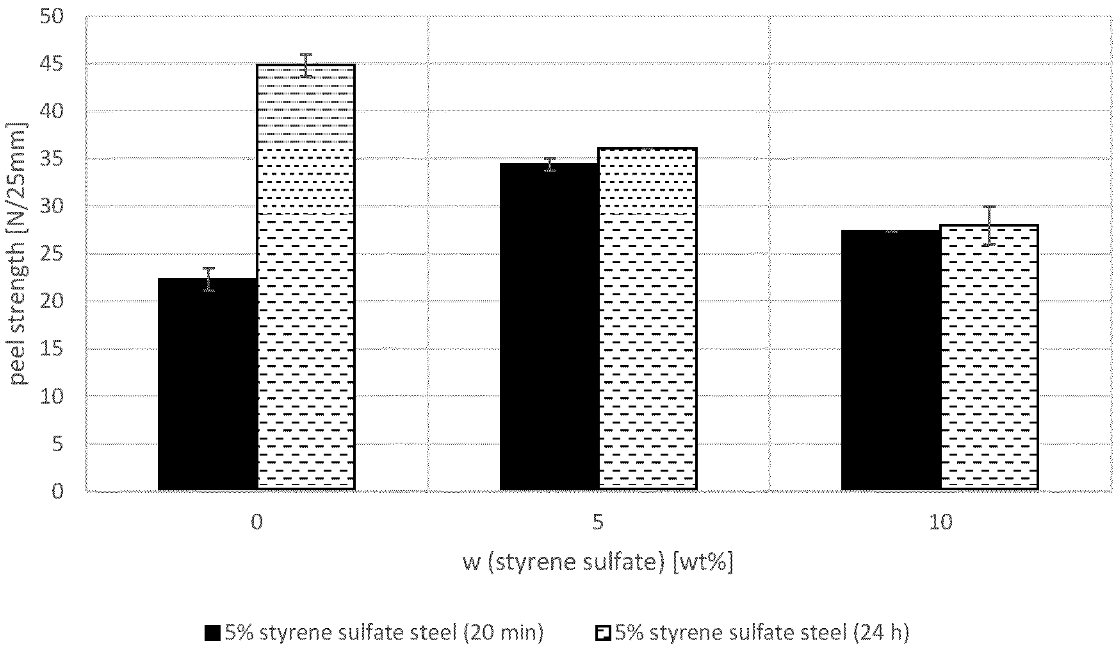
50
45
40
35
30
25
20
15
10
5
0
peel strength [N/25mm]
0
5
10
w (styrene sulfate) [wt%]
5% styrene sulfate steel (20 min)
5% styrene sulfate steel (24 h)

Figure 3b

Europäisches Patentamt
European Patent Office
Office européen des brevets

# EUROPEAN SEARCH REPORT

Application Number
EP 22 19 2188

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | CN 107 973 874 B (UNIV BEIJING) 2 June 2020 (2020-06-02)<br>* abstract *<br>* paragraphs [0010], [0028] * | 1,15<br>1-15 | INV.<br>C08F220/18<br>C09J133/06 |
| X<br>Y | EP 0 869 979 B1 (MINNESOTA MINING & MFG [US]) 8 November 2000 (2000-11-08)<br>* paragraphs [0001], [0005] - [0007], [0012], [0016] - [0017], [0028] * | 1-4,15<br>1-15 | |
| Y | EP 3 964 257 A1 (HENKEL AG & CO KGAA [DE]) 9 March 2022 (2022-03-09)<br>* the whole document * | 1-15 | |
| Y | WO 2020/178218 A1 (HENKEL AG & CO KGAA [DE]) 10 September 2020 (2020-09-10)<br>* the whole document * | 1-15 | |
| Y | CN 111 117 533 B (WUXI TAMAY NEW MAT CO LTD) 7 December 2021 (2021-12-07)<br>* abstract *<br>* paragraph [0016]; example 1 * | 1-15 | TECHNICAL FIELDS SEARCHED (IPC) |
| Y | US 2014/211374 A1 (SUGIHARA RYOSUKE [JP] ET AL) 31 July 2014 (2014-07-31)<br>* paragraphs [0043], [0049]; examples * | 1-15 | C08F<br>C09J |

The present search report has been drawn up for all claims

1

| Place of search | Date of completion of the search | Examiner |
|---|---|---|
| The Hague | 9 February 2023 | Droghetti, Anna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 22 19 2188

This annex lists the patent family members relating to the patent documents cited in the above-mentioned European search report. The members are as contained in the European Patent Office EDP file on
The European Patent Office is in no way liable for these particulars which are merely given for the purpose of information.

09-02-2023

| Patent document cited in search report | Publication date | Patent family member(s) | Publication date |
|---|---|---|---|
| CN 107973874 B | 02-06-2020 | NONE | |
| EP 0869979 B1 | 08-11-2000 | AU 4744896 A | 28-07-1997 |
| | | DE 69519395 T2 | 13-06-2001 |
| | | EP 0869979 A1 | 14-10-1998 |
| | | JP 2000503054 A | 14-03-2000 |
| | | WO 9724378 A1 | 10-07-1997 |
| EP 3964257 A1 | 09-03-2022 | EP 3964257 A1 | 09-03-2022 |
| | | TW 202212520 A | 01-04-2022 |
| | | WO 2022048910 A1 | 10-03-2022 |
| WO 2020178218 A1 | 10-09-2020 | CA 3131757 A1 | 10-09-2020 |
| | | CN 113557281 A | 26-10-2021 |
| | | EP 3935119 A1 | 12-01-2022 |
| | | TW 202039757 A | 01-11-2020 |
| | | US 2022098454 A1 | 31-03-2022 |
| | | WO 2020178218 A1 | 10-09-2020 |
| CN 111117533 B | 07-12-2021 | NONE | |
| US 2014211374 A1 | 31-07-2014 | CN 103748164 A | 23-04-2014 |
| | | CN 105348667 A | 24-02-2016 |
| | | JP 5252669 B1 | 31-07-2013 |
| | | JP 5281209 B1 | 04-09-2013 |
| | | JP 2013177588 A | 09-09-2013 |
| | | JP WO2013035548 A1 | 23-03-2015 |
| | | KR 20140057490 A | 13-05-2014 |
| | | KR 20160034431 A | 29-03-2016 |
| | | TW 201315769 A | 16-04-2013 |
| | | TW 201629149 A | 16-08-2016 |
| | | US 2014211374 A1 | 31-07-2014 |
| | | US 2017025227 A1 | 26-01-2017 |
| | | WO 2013035548 A1 | 14-03-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2018086948 A
- WO 2020178218 A